# EUROPÄISCHE PATENTSCHRIFT

(11) **EP 4 406 588 B1**
(45) Veröffentlichungstag und Bekanntmachung des Hinweises auf die Patenterteilung: **07.10.2026**
(21) Anmeldenummer: 24167297.1
(22) Anmeldetag: 29.01.2021
(51) Int. Cl.: A61N 1/04

(54) **UNTERBEKLEIDUNGSSTÜCK ZUM TRAGEN UNTER EINEM ELEKTROMUSKELSTIMULATIONSKLEIDUNGSSTÜCK**
UNDERGARMENT FOR SUPPORT UNDER AN ELECTRIC MUSCLE STIMULATION GARMENT
SOUS-VÊTEMENT DESTINÉ À ÊTRE PORTÉ SOUS UN VÊTEMENT DE STIMULATION ÉLECTROMUSCULAIRE

(30) Priorität: 30.01.2020 DE 202020100505 U
(43) Veröffentlichungstag der Anmeldung: 31.07.2024
(62) Dokumentnummer(n) der früheren Anmeldung(en) nach Art. 76 EPÜ: 21154193.3 / 3 858 423
(73) Patentinhaber: XBody Hungary Kft., 1125 Budapest (HU)
(72) Erfinder: Füzessy, Balàzs, 9171 Gyôrùjfalu (HU)
(74) Vertreter: Grünecker Patent- und Rechtsanwälte PartG mbB

(56) Entgegenhaltungen:
- DE-A1- 10 248 235
- DE-A1- 102009 013 627
- DE-A1- 102012 112 153
- DE-A1- 102014 108 316
- DE-A1- 102015 118 837
- DE-U1- 202014 103 548

## Beschreibung

Die vorliegende Erfindung betrifft ein Unterbekleidungsstück zum Tragen unter einem Elektromuskelstimulations-Kleidungsstück mit Merkmalen aus dem Anspruch 1 sowie eine zugehörige Kombination aus einem solchen Unterbekleidungsstück mit einem Elektromuskelstimulations-Kleidungsstück, und eine zugehörige Verwendung.

Elektromuskelstimulation oder auch Elektromyostimulation (kurz: EMS) findet in vielen Bereichen der sportlichen Leistungsverbesserung und Leistungserhaltung sowie in körperlichen Rehabilitationstherapien eine Anwendung. Die Teile, welche bei einer EMS-Anwendung mit dem Körper, insbesondere mit dem Körperschweiß des EMS-Kleidungsstück-Trägers/EMS-Anwendungsempfängers, in Berührung kommen, müssen aus Hygienegründen nach jeder Anwendung gereinigt werden.

Aus der DE 10 2009 013 627 A1 ist ein EMS-Bekleidungsstück bekannt, welches aus einem Innenbekleidungsstück und einem Außenbekleidungsstück besteht. Die beiden Bekleidungsstücke sind voneinander trennbar, so dass das Innenbekleidungsstück leichter gereinigt oder ausgetauscht werden kann. Bei diesem System sind die die Haut kontaktierenden Muskelstimulationselektroden an der Innenseite des Innenbekleidungsstücks vorgesehen und die Elektroden-Anschlussstecker und Elektrodenverbindungselemente auf dem Außenbekleidungsstück vorgesehen. Von der Innenseite des Innenbekleidungsstücks ragen stiftartige Kontakte durch das Innenbekleidungsstück zur Außenseite des Innenbekleidungsstücks, wo sie jeweils mit entsprechenden Verbindungssteckern des Außenbekleidungsstücks manuell zu verbinden sind.

Bei derartigen EMS-Bekleidungsstücken kann es jedoch zu einer unerwünschten Druckstellenbildung, insbesondere im Bereich der stiftartigen Durchkontaktierung, kommen, was zu Tragekomforteinbußen führt. Weiterhin sind derartige EMS-Bekleidungsstücke zeitaufwändig in der Handhabung, da jede Elektrode vor der EMS-Anwendung manuell mit dem jeweils zugehörigen Stecker des Außenbekleidungsstücks verbunden werden muss und nach der Anwendung wieder gelöst werden muss.

Der vorliegenden Erfindung liegt die Aufgabe zugrunde EMS Anwendungen für den Anwendungsempfänger komfortabler zu machen.

Die Erfindung löst diese Aufgabe mit einem Unterbekleidungsstück zum Tragen unter einem Elektromuskelstimulations-Kleidungsstück mit den Merkmalen gemäß Anspruch 1.

Das Unterbekleidungsstück weist hierbei mindestens einen sich durch das Unterbekleidungsmaterial erstreckenden, elektrisch leitfähigen Durchleitungsabschnitt auf, welcher eine Elektroimpulsempfangsfläche auf der körperabgewandten Unterbekleidungsmaterial-Außenseite und eine Elektroimpulsabgabefläche auf der körperzugewandten Unterbekleidungsmaterial-Innenseite umfasst, und der dazu eingerichtet ist, elektrische Impulse von der Elektroimpulsempfangsfläche zur Elektroimpulsabgabefläche zu leiten, wobei der mindestens eine Durchleitungsabschnitt in dem Unterbekleidungsmaterial flächig und einstückig integriert ist.

Durch das Vorsehen einer Elektroimpulsempfangsfläche auf der Unterbekleidungsaußenseite zusammen mit einer flächigen und einstückigen Integration des gesamten Durchleitungsabschnitts in das Unterbekleidungsmaterial, kann eine Druckstellenbildung in diesem Bereich reduziert und der Tragekomfort erhöht werden.

Unter "flächig und einstückig integriert" ist hier eine dauerhafte und nicht-zerstörungsfreilösbare Verbindung des mindestens einen Durchleitungsabschnitts in das restliche, den Durchleitungsabschnitt umgebende Unterbekleidungsmaterial zu verstehen. Der mindestens eine Durchleitungsabschnitt ist dabei ein integraler Bestandteil des Unterbekleidungsmaterials und bildet mit dem umgebenden Unterbekleidungsmaterial in der Fläche eine durchgehende Einheit.

Auch wird es durch die Elektroimpulsempfangs- und Elektroimpulsabgabefläche ermöglicht, elektrischen Impulse von einem EMS-Kleidungsstück in den Durchleitungsabschnitt und letztendlich auf die Haut mittels flexibleren elektrischen Verbindungen zu übertragen, beispielsweise durch ein rein flächiges Anliegen der Muskelstimulationselektroden eines EMS-Kleidungsstücks an die Elektroimpulsempfangsflächen der Durchleitungsabschnitte.

So kann das erfindungsgemäße Unterbekleidungsstück sehr komfortabel, beispielsweise wie eine textile Ski-Unterwäsche, unter einem EMS-Kleidungsstück getragen werden, wobei die Muskelstimulationselektroden des EMS-Kleidungsstücks beim Festziehen des EMS-Kleidungsstücks an die Elektroimpulsempfangsflächen gedrückt werden. Somit wird eine Elektroimpulsübertragung sichergestellt, ohne dass eine steife, strukturelle Verbindung zwischen den beiden Kleidungsstücken notwendig wird.

Hierdurch wird es zudem bis zu einem gewissen Grad möglich, dass ein elektrischer Impuls trotz einer leichten Relativbewegung zwischen Elektroimpulsempfangsfläche und Muskelstimulationselektrode des EMS-Kleidungsstücks noch durchgeleitet wird. Zudem kann das erfindungsgemäße Unterbekleidungsstück beispielweise mit bestehenden EMS-Kleidungsstücken benutzt werden, welche flächige Muskelstimulationselektroden zum direkten Kontakt mit der Haut aufweisen.

Die einstückig flächige Integrierung des mindestens einen Durchleitungsabschnitts in das Unterbekleidungsmaterial erlaubt es weiterhin, das Unterbekleidungsmaterial einschließlich des mindestens einen Durchleitungsabschnitt in gewissen Ausführungsformen in einem Fertigungsverfahren herzustellen, beispielsweise wenn der mindestens eine Durchleitungsabschnitt in das Unterbekleidungsmaterial eingewebtes, eingestricktes oder eingewirktes elektrisch leitfähiges Garn aufweist.

In einer möglichen Ausführungsform können sich die Elektroimpulsempfangsfläche und die Elektroimpulsabgabefläche in Größe und/oder in Materialbeschaffenheit einander entsprechen.

Durch eine gleiche flächige Ausdehnung der Elektroimpulsempfangsfläche und der Elektroimpulsabgabefläche kann der elektrische Impuls gleichmäßig durch das Unterbekleidungsmaterial geleitetet werden. Auch wird es hierdurch möglich, bei einer von außen sichtbaren Elektroimpulsempfangsfläche auf die Ausdehnung der inneren Elektroimpulsabgabefläche zu schließen. Sich optional einander entsprechende Materialbeschaffenheit bei beiden Flächen ermöglichen es, beide Flächen mit derselben Technik zu fertigen und beispielsweise den gesamten Durchleitungsabschnitt mit dem gleichen Material auszuführen und mit einer einzigen Herstellungs-Technik zu fertigen.

In einer weiteren möglichen Ausführung können die Elektroimpulsempfangsfläche und/oder die Elektroimpulsabgabefläche mit dem angrenzenden Unterbekleidungsmaterial oberflächenbündig sein, und/oder das Unterbekleidungsmaterial zumindest im Bereich des mindestens einen Durchleitungsabschnitts und dem angrenzenden Unterbekleidungsmaterial einen konstanten Querschnitt aufweisen.

Eine solch oberflächenbündige Ausführung reduziert ein Reiben besagter Stellen an der Haut des Trägers oder am EMS-Kleidungsstück und erhöht so weiter den Tragekomfort, selbst wenn noch eine zusätzliche elektrisch leitfähige Beschichtung auf die Flächen aufgetragen sein sollte. Dasselbe gilt für einen optionalen konstanten Querschnitt durch das Unterkleidungsmaterial, sowohl an dem mindestens einen Durchleitungsabschnitt als auch an den anderen, angrenzenden Bereichen des Unterbekleidungsmaterials. In einer möglichen Ausführung kann auch das Unterbekleidungsmaterial des gesamten Unterbekleidungsstücks einen durchgehend konstanten Materialquerschnitt aufweisen. Gleichzeitig kann bei solchen Ausführungen beispielsweise auf effiziente Fertigungstechniken zurückgegriffen werden, welche einen konstanten Querschnitt oder konstant glatte Materialoberflächen herstellen oder voraussetzen.

Optional können der oder die Durchleitungsabschnitte so im Unterbekleidungsstück angeordnet sein, dass die zugehörigen Elektroimpulsabgabeflächen beim ordnungsgemäßen Tragen des Unterbekleidungsstücks an einer oder mehreren Muskelpartien des Trägers sitzen, insbesondere an einer oder mehreren Muskelpartien der Skelettmuskulatur des Trägers sitzen, wie beispielsweise an Muskelpartien des Rückens, der Brust, der Beine, der Arme, des Bauchs und/oder der Schultern.

Dies erlaubt es beispielsweise, je nach Ausführung des Unterbekleidungsstücks, verschiedene Muskelpartien des Trägers für ein EMS-Kleidungsstück zugänglich zu machen oder unzugänglich zu machen. Weiterhin können spezielle Muskelpartien gezielter örtlich angesprochen werden, als dies mit einem EMS-Kleidungsstück ohne Einsatz eines erfindungsgemäßen Unterbekleidungsstücks möglich wäre, da das Unterbekleidungsstück vergleichsweise einfach nach den individuellen Wünschen und Gegebenheiten eines Trägers anfertigbar ist. Dies erhöht den Tragekomfort weiter.

Es ist ebenfalls denkbar das in einer möglichen Ausführung der Erfindung der mindestens eine Durchleitungsabschnitt eine vergleichbare Flexibilität wie das angrenzende Unterbekleidungsmaterial aufweist.

Hierdurch werden Druckstellen weiter reduziert und der Tragekomfort weiter erhöht. Die Durchleitungsabschnitte können sich hierbei an die individuelle Kontur des Trägers anpassen und zeigen ein vergleichbares Anschmiegeverhalten wie das restliche Unterbekleidungsmaterial.

In einer weiteren möglichen Ausführungsform kann das Unterbekleidungsstück ein Oberkörperbekleidungsstück und/oder ein Unterkörperbekleidungsstück aufweisen.

Hierdurch kann in vertrauter Weise das Unterbekleidungsstück am Oberkörper, beispielweise in Form eines Shirts oder einer Weste, und/oder am Unterkörper, beispielsweise in Form einer Hose oder Kniestrümpfen, getragen werden und die entsprechenden Muskelpartien können durch die Durchleitungsabschnitte verlässlich angesprochen werden.

In einer ebenfalls möglichen, weiteren Ausführungsform ist das Unterbekleidungsstück kabellos ausgeführt und/oder weist keine feste elektrische Kontaktstruktur, wie beispielsweise einen Kontaktstecker, einen Kontaktstift und/oder eine feste Durchkontaktierung, auf.

Der Tragekomfort sowie die Handhabung des Unterbekleidungsstücks werden hierdurch weiter erhöht.

In einer vorteilhaften Variante der Erfindung kann der mindestens eine Durchleitungsabschnitt als Trockenelektrode ausgeführt sein.

Hierdurch ist unabhängig von der Hautfeuchtigkeit und der Transpiration des Trägers eine stets komfortable Elektroimpulsabgabe in die Haut und somit den Muskel des Trägers gewährleistet. Ein anfängliches Anfeuchten der Elektroimpulsabgabefläche ist nicht notwendig. Erfindungsgemäß weist das Unterbekleidungsmaterial zumindest im Bereich des mindestens einen Durchleitungsabschnitts und dem angrenzenden Unterbekleidungsmaterial einen gemäß demselben Herstellungsverfahren gefertigten textilen Stoff auf. Optional kann das Unterbekleidungsmaterial durchgehend einen gemäß demselben Herstellungsverfahren gefertigten textilen Stoff aufweisen.

Hierdurch kann ein hoher und homogener Tragekomfort an Stellen, insbesondere an allen Stellen, des Unterbekleidungsstücks, einschließlich des mindestens einen Durchleitungsabschnitts, erreicht werden. Weiterhin können sowohl Durchleitungsabschnitte als auch andere Bereiche des Unterbekleidungsmaterials aus derselben oder gleichartigen textilen Stoffbahnen gefertigt werden oder zumindest das gleiche Textil als Trägermaterial aufweisen, was Vorteile in der Produktion mit sich bringt. Optional kann das gesamte Unterbekleidungsmaterial des Unterbekleidungsstücks einen gemäß demselben Herstellungsverfahren gefertigten textilen Stoff aufweisen. Erfindungsgemäß weist der mindestens eine Durchleitungsabschnitt in das Unterbekleidungsmaterial eingenähtes elektrisch leitfähiges Garn auf.

Der mindestens eine Durchleitungsabschnitt weist in das Unterbekleidungsmaterial eingenähtes elektrisch leitfähiges Garn auf.

Der Einsatz von elektrisch leitfähigen Fasern ermöglicht eine zuverlässige elektrische Leitfähigkeit, wobei durch eine mögliche Kombination mit einem flexiblen Trägermaterial trotzdem ein hoher Tragekomfort erreicht werden kann. Durch den Einsatz von elektrisch leitfähigem Garn ist es möglich, auf erprobte Verfahren aus der textilen Massenproduktion zurückzugreifen. Für die Durchleitungsabschnitte mit eingenähten elektrisch leitfähigen Garnen kann bei der Herstellung auf diese besonders erprobten Fertigungsverfahren zurückgegriffen werden. Die Durchleitungsabschnitte werden hierbei in das Unterbekleidungsmaterial flächig und einstückig integriert und ein hoher Tragekomfort wird ermöglicht.

In einer weiteren optionalen Ausführung kann der mindestens eine Durchleitungsabschnitt ein elektrisch leitfähiges Polymer, insbesondere ein elektrisch leitfähiges Silikon, aufweisen, insbesondere in Kombination mit einem textilen Trägermaterial.

Durch den Einsatz eines elektrisch leitfähigen Polymers kann ebenfalls ein hoher Tragekomfort erreicht werden und die Durchleitungsabschnitte in das Unterbekleidungsmaterial flächig und einstückig integriert werden. Der optionale Einsatz von elektrisch leitfähigem Silikon ist hierbei besonders vorteilhaft. Weiterhin ist es möglich das elektrisch leitfähige Polymer, insbesondere das Silikon, mit einem textilen Trägermaterial zu kombinieren, um einen hohen Tragekomfort an den Durchleitungsabschnitten zu gewährleisten.

In einer weiteren möglichen Variante der Erfindung kann die Elektroimpulsabgabefläche und/oder die Elektroimpulsempfangsfläche mit einem elektrisch leitfähigen Medium, insbesondere mit einem elektrisch leitfähigen Polymer, beispielsweise einem elektrisch leitfähigen Silikon oder einem elektrisch leitfähigen Polyurethan, beschichtet sein.

Eine solche Beschichtung verbessert dauerhaft die Weiterleitung des elektrischen Impulses von der Elektroimpulsabgabefläche in die Haut und folglich in den Muskel des Trägers, auch wenn die Kontaktfläche zur Haut trocken sein sollte. Eine homogene Impulsweiterleitung wird hierdurch auch bei trockener Haut möglich, was Spannungsspitzen reduziert und den Tragekomfort verbessert. Elektrisch leitfähige Polymere, insbesondere elektrisch leitfähige Silikone und elektrisch leitfähiges Polyurethan, haben sich hier als besonders geeignet herausgestellt. Bei der Elektroimpulsempfangsfläche kann eine solche Beschichtung Verschleiß durch Abrieb reduzieren.

Eine weitere mögliche Ausführungsform der Erfindung betrifft eine Kombination aufweisend ein Unterbekleidungsstück nach einer der vorherigen Ausführungen und ein Elektromuskelstimulations-Kleidungsstück mit mindestens einer Muskelstimulations-Elektrode.

Bei einer solchen Kombination können Unterbekleidungsstück und EMS-Kleidungsstück optimal aufeinander abgestimmt werden.

In einer weiteren möglichen Ausführungsform ist das zuvor genannte Elektromuskelstimulations-Kleidungsstück an einer Stelle vollkommen öffenbar und wiederverschließbar, beispielweise durch einen Reißverschluss und/oder Haken-, Schlaufen- oder Gurtsystemen. Zudem kann das Elektromuskelstimulations-Kleidungsstück Justiersysteme, beispielsweise Festziehgurte, aufweisen, um den Sitz und den Anliegedruck des EMS-Kleidungsstücks an bestimmten Körperstellen zu verändern.

In einer besonders vorteilhaften optionalen Ausführung der Kombination können die Position eines Durchleitungsabschnitts des Unterbekleidungsstücks und die Position einer Muskelstimulations-Elektrode des Elektromuskelstimulations-Kleidungsstücks so aufeinander abgestimmt sein, dass beim Tragen des Unterbekleidungsstücks mit dem darüberliegenden Elektromuskelstimulations-Kleidungsstück die Muskelstimulations-Elektrode an einer Elektroimpulsempfangsfläche des Durchleitungsabschnitts anliegt, insbesondere jede Muskelstimulations-Elektrode an einer Elektroimpulsempfangsfläche anliegt.

Durch ein derartiges Aufeinanderabstimmen der Positionen von Durchleitungsabschnitt und Muskelstimulationselektrode des EMS-Kleidungsstücks wird eine zuverlässige Impulsweiterleitung von der Muskelstimulationselektrode über den jeweiligen Durchführungsabschnitt in die Haut und somit in den Muskel des Trägers sichergestellt.

In einer weiteren erfindungsgemäßen Kombination ist es möglich, die flächige Ausdehnung der Elektroimpulsempfangsfläche gleich groß oder größer als die flächige Ausdehnung der zugehörigen Muskelstimulations-Elektrode zu gestalten.

Hierdurch ist eine besonders zuverlässige Impulsweiterleitung vom EMS-Kleidungsstück durch den jeweiligen Durchleitungsabschnitt sichergestellt, auch wenn es zu kleineren Relativbewegungen zwischen Muskelstimulations-Elektrode und zugehöriger Elektroimpulsempfangsfläche kommt.

Eine weitere mögliche Ausführungsform der Erfindung betrifft eine Verwendung eines hier zuvor beschriebenen Unterbekleidungsstücks zum Tragen unter einem Elektromuskelstimulations-Kleidungsstück, insbesondere während einer nicht-therapeutischen Elektromuskelstimulations-Anwendung, beispielsweise in einem Fitness-Studio oder in anderen nicht-therapeutischen und nicht-medizinischen Einrichtungen oder Wirkstätten, sowie zuhause.

Wirkungen und Vorteile zu einer solchen Verwendung werden aus den vorherigen Erläuterungen zu den einzelnen möglichen Ausführungsformen des Unterbekleidungsstücks deutlich. Das Unterbekleidungsstück kann beispielsweise mit gängigen EMS-Kleidungsstücken, welche gängige Hautkontakt-Muskelstimulations-Elektroden aufweisen, verwendet werden, ohne derartige EMS-Kleidungsstücke dabei speziell hierfür modifiziert werden müssten.

Eine weitere mögliche Variante der Erfindung betrifft die Verwendung einer hier zuvor erläuterten Kombination von EMS-Kleidungsstück und Unterbekleidungsstück während einer nicht-therapeutischen Elektromuskelstimulations-Anwendung, beispielsweise in einem Fitness-Studio oder in anderen nicht-therapeutischen und nicht-medizinischen Einrichtungen oder Wirkstätten, sowie zuhause. Insbesondere die Verwendung beider Komponenten in Kombination ermöglicht zahlreiche Verbesserungen für den Träger währen einer EMS-Anwendung, wie den vorherigen Erläuterungen entnommen werden kann.

Die hier offenbarten nicht-therapeutischen Elektromuskelstimulationsanwendungen dienen insbesondere der rein sportlichen Leistungsverbesserung und/oder insbesondere der rein sportlichen Leistungserhaltung.

Nachfolgen wird zum leichteren Verständnis der Erfindung eine mögliche Ausführungsform der Erfindung anhand der Figuren näher erläutert. Diese Ausführungsform zeigt hierbei eine mögliche Kombination von zuvor genannten Erfindungsmerkmalen. Das Dokument DE-A-10 2012 112153 offenbart ein Unterbekleidungsstück gemäß Oberbegriff des Anspruchs 1. Dabei zeigen
- Figur 1: eine schematische Ansicht von Teilen eines Trägers, welcher ein erfindungsgemäßes Unterbekleidungsstück, hier in der Form einer Oberkörperbekleidung und einer Unterkörperbekleidung, trägt,
- Figur 2: eine schematische Querschnittsansicht durch ein erfindungsgemäßes Unterbekleidungsmaterial samt Durchleitungsabschnitt,
- Figur 3: eine schematische Ansicht eines Elektromuskelstimulations-Kleidungsstücks mit einem darunterliegenden erfindungsgemäßen Unterbekleidungsstück an einem schematisch dargestellten Träger,
- Figur 4: eine Ansicht von Innen auf ein Elektromuskelstimulations-Kleidungsstück in Form einer Weste,
- Figur 5: eine schematische Querschnittsansicht im Bereich eines Durchleitungsabschnitts eines Unterbekleidungsstücks, wobei das Unterbekleidungsstück unter einem Elektromuskelstimulations-Kleidungsstück und auf der Haut eines Trägers getragen wird.

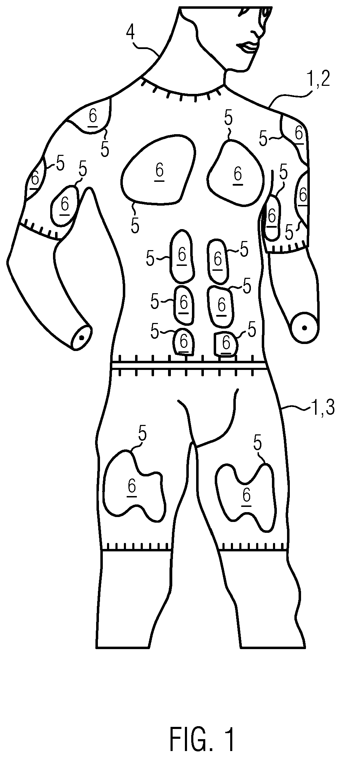

Figur 1 zeigt ein schematisch dargestelltes Unterbekleidungsstück 1, hier zweiteilig ausgeführt als Oberkörperbekleidungsstück 2 in Form eines eng anliegenden T-Shirts und als separates Unterkörperbekleidungsstück 3 in Form einer engen anliegenden kurzen Hose. Das Unterbekleidungsstück 1 wird hier von einem schematisch dargestellten Träger 4, also einem EMS-Anwendungsempfänger, getragen. Das Unterbekleidungsstück 1 ist flexibel und hier auch elastisch ausgeführt.

Das Unterbekleidungsstück 1 weißt hierbei an Stellen derjenigen Skelettmuskelpartien des Trägers, welche Elektrostimulation erfahren sollen, zahlreiche Durchleitungsabschnitte 5 auf. Der Betrachter sieht hier die dem Träger 4 abgewandte Elektroimpulsempfangsflächen 6 der jeweiligen Durchleitungsabschnitte 5.

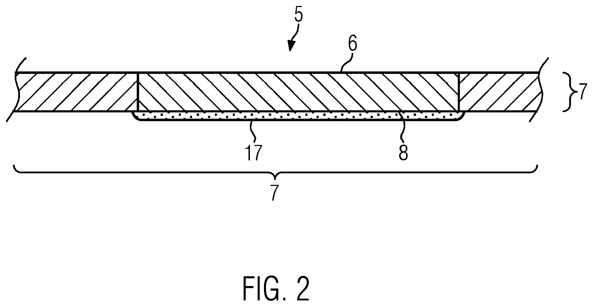

Figur 2 zeigt eine schematische Querschnittsansicht durch einen der Durchleitungsabschnitte 5 zusammen mit angrenzendem Unterbekleidungsmaterial 7. Der Durchleitungsabschnitt 5 ist Teil des Unterbekleidungsmaterials 7.

Wie der Figur 2 entnehmbar ist, ist der elektrisch leitfähige Durchleitungsabschnitt 5 flächig und einstückig in dem Unterbekleidungsmaterial 7 integriert. Die Materialstärke des Durchleitungsabschnitts 5 und des restlichen Unterbekleidungsmaterials 7 ist im Querschnitt durchgehend konstant.

Auf der beim Tragen körperabgewandten Außenseite des Unterbekleidungsmaterials 7 weist der Durchleitungsabschnitt 5 eine Elektroimpulsempfangsfläche 6 auf und auf der beim Tragen körperzugewandten Innenseite des Unterbekleidungsmaterials 7 weist der Durchleitungsabschnitt 5 eine Elektroimpulsabgabefläche 8 auf. Die Elektroimpulsabgabefläche 8, und somit das Unterbekleidungsmaterial 7 an dieser Stelle, weist in dieser Ausführung eine sich über die gesamte Elektroimpulsabgabefläche 8 erstreckende elektrisch leitfähige Beschichtung 17 auf.

Hier sind Elektroimpulsempfangsfläche 6 und Elektroimpulsabgabefläche 8 gleich groß, entsprechen sich also in ihrer hier horizontalen flächigen Erstreckung. Weiterhin weisen hier beide Flächen 6, 8, sowie hier auch der Materialbereich zwischen den beiden Flächen 6, 8, dieselbe Materialbeschaffenheit auf und sind in dieser Ausführung aus demselben Material oder denselben Materialkomponenten gefertigt, und haben vergleichbare physikalische Eigenschaften.

Die Elektroimpulsempfangsfläche 6 und die Elektroimpulsabgabefläche 8 sind in dieser Ausführungsform mit dem angrenzenden Unterbekleidungsmaterial oberflächenbündig ausgeführt, was in der Figur 2 gut zu sehen ist.

Der Durchleitungsabschnitt 5 hat hier eine vergleichbare Flexibilität wie das angrenzende Unterbekleidungsmaterial.

Der Durchleitungsabschnitt 5 ist hier als Trockenelektrode ausgeführt, wobei in dieser Ausführungsform, die nicht Teil der Erfindung ist, ein nicht dargestelltes elektrisch leitfähiges Garn, beispielsweise ein ein Metall enthaltendes Garn, bereits im ursprünglichen Herstellungsprozess des textilen Unterbekleidungsmaterials 7 mit in die gestrickte textile Struktur hineingestrickt wurde.

In weiteren Ausführungsformen, die nicht Teil der Erfindung sind, kann der Durchleitungsabschnitt 5 elektrisch leitendes Garn aufweisen, welches bereits im ursprünglichen textilen Herstellungsprozess des Unterbekleidungsmaterials 7 in das gewebte oder gewirkte Unterbekleidungsmaterial 7 mit eingewebt oder mit eingewirkt wurde.

Der Durchleitungsabschnitt 5 weist elektrisch leitendes Garn auf, welches nach der ursprünglichen textilen Herstellung des Unterbekleidungsmaterials 7 in das Unterbekleidungsmaterial 7 eingenäht, also eingestickt, wurde.

In weiteren möglichen Ausführungsformen ist elektrisch leitfähiges Garn derart in das Unterbekleidungsmaterial eingenäht bzw. eingestickt, dass das leitfähige Garn flächenmäßig zumindest den Großteil, insbesondere die Gesamtheit, der Elektroimpulsempfangsfläche 6 und/oder der Elektroimpulsabgabefläche 8 bildet.

Die zuvor genannten elektrisch leitfähigen Garne durchziehen hierbei den Durchleitungsabschnitt 5 von der Elektroimpulsempfangsfläche 6 bis zur Elektroimpulsabgabefläche 8.

In einer weiteren Ausführungsform enthält der Durchleitungsabschnitt 5 ein elektrisch leitfähiges Silikon, beispielsweise in Kombination mit einem textilen Trägermaterial.

Die elektrisch leitfähige Beschichtung 17 der Elektroimpulsabgabefläche 8 weist in dieser Ausführungsform elektrisch leitfähiges Polyurethan auf, um die Elektroimpulsweitergabe auch bei nicht angefeuchteter Haut zu gewährleisten. Die Materialdicke des Unterbekleidungsmaterials 7 und insbesondere des Durchleitungsabschnitts 5 liegt in möglichen Ausführungsformen im Bereich von 0,3 mm bis 10 mm, insbesondere im Bereich von 0,8 mm bis 2,5 mm. Die Dicke der Beschichtung 17, welche beispielsweise in Form einer zusätzlichen Lage fest aufgebracht, insbesondere aufgeklebt, sein kann, liegt in möglichen Ausführungen im Bereich zwischen 0 mm und 4 mm, insbesondere im Bereich von 0,2 mm bis 3,5 mm. Beispielsweise bei leitfähigem Polyurethan liegt die Schichtdicke im Bereich zwischen 0 mm und 2 mm, insbesondere im Bereich von 0,1 mm bis 1 mm. Beispielsweise bei leitfähigem Silikon liegt die Schichtdicke im Bereich von 1 mm bis 3,5 mm, insbesondere im Bereich von 1,5 mm bis 3 mm.

In möglichen Ausführungsformen besteht das hier offenbarte Unterbekleidungsmaterial zumindest teilweise aus einer Kombination von flachgestricktem Polyester (PES) und Elastan.

Das Unterbekleidungsmaterial 7 kann in weiteren möglichen Ausführungsformen im Bereich der Elektroimpulsempfangsfläche 6 ebenfalls mit einer zuvor genannten elektrisch leitfähigen Beschichtung versehen sein. Eine derartige Beschichtung der Elektroimpulsempfangsfläche 6 ist jedoch in den Figuren nicht dargestellt.

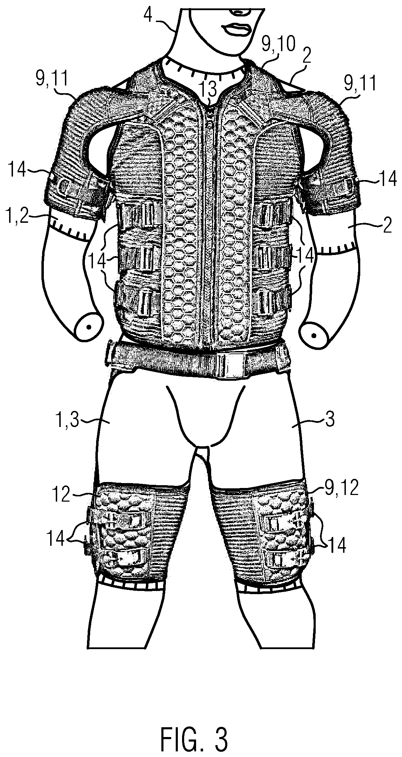

Figur 3 zeigt eine Kombination aus dem Unterbekleidungsstück 1 und einem Elektromuskelstimulations-Kleidungsstück 9 (kurz: EMS-Kleidungsstück), welches in dieser Ausführung eine EMS-Weste 10, zwei separate EMS-Schulterteile 11 und eine separate EMS-Hose 12 umfasst. Die EMS-Hose 12 bedeckt auf der Vorderseite des Trägers 4 nur die untere Hälfte der Oberschenkel. Alle EMS-Kleidungsteile 10-12 sind in dieser Ausführung am Rücken des Trägers 4 mit einer Stromversorgungs- und Steuereinheit verkabelt. In dieser Ausführung kann jedes Teil 10-12 des EMS-Kleidungsstücks 9 an einer Stelle vollständig geöffnet und wiederverschlossen werden, beispielsweise durch einen Reißverschluss 13 oder andere Verschlusssysteme. Justiersysteme, hier in Form von Zuggurten 14, erlauben ein Justieren des Sitzes und ein Festziehen der einzelnen EMS-Kleidungsstück-Teile 10-12 am Körper.

Das EMS-Kleidungsstück 9 weist hierbei auf seiner Innenseite Muskelstimulationselektroden 15 auf. Die Muskelstimulationselektroden 15 sind in dieser Ausführung Hautkontakt-Elektroden, welche auch in direktem Hautkontakt mit dem Träger 4 ihre vorgesehene Funktion erfüllen würden. Die Positionen der einzelnen Muskelstimulationselektroden 15 des EMS-Kleidungsstücks 9 korrespondieren hierbei mit den Positionen der einzelnen Durchleitungsabschnitte 5 auf dem Unterbekleidungsstück 1.

In anderen Ausführungsformen ist es möglich, dass die Anzahl der einzelnen Durchleitungsabschnitte 5 nicht mit der Anzahl der Muskelstimulationselektroden 15 des EMS-Kleidungsstücks 9 korrespondiert, beispielsweise wenn das hier gezeigte Oberkörperbekleidungsstück 2 lediglich mit einer EMS-Weste 10 genutzt wird, so dass die Durchleitungsabschnitte 5 an den Schultern und den Oberarmen bei der Anwendung ungenutzt bleiben.

In dieser Ausführung sind die jeweiligen Elektroimpulsempfangsflächen 6 der einzelnen Durchleitungsabschnitte 5 in ihrer flächigen Ausdehnung in etwa gleich groß wie die flächige Ausdehnung der Impulsübertragungsflächen 16 der korrespondierenden Muskelstimulationselektroden 15 des EMS-Kleidungsstücks 9.

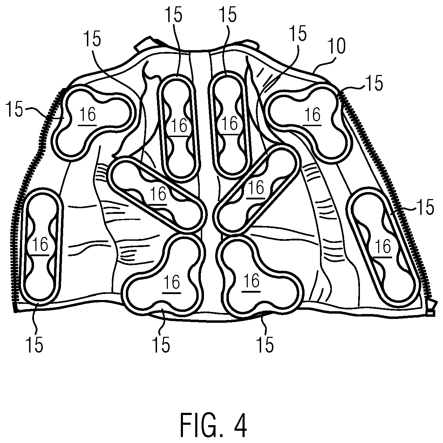

In Figur 4 ist die Innenseite der EMS-Weste 10 gezeigt. Hierbei sind links und rechts die Muskelstimulationselektroden 15 zu sehen, welche beim Tragen der EMS-Weste 10 an der Vorderseite des Oberkörperbekleidungsstücks 2 an den jeweiligen Elektroimpulsempfangsflächen 6 der entsprechenden Durchleitungsabschnitte 5 der Brustmuskel- und Bauchmuskelpartien des Trägers 4 anliegen. Beispielsweise durch das Festziehen einzelner Zuggurte 14 an der Weste 10 kann ein sitzfestes Anliegen der einzelnen Muskelstimulationselektroden 15 an den jeweiligen Elektroimpulsempfangsflächen 6 während einer EMS-Anwendung sichergestellt werden.

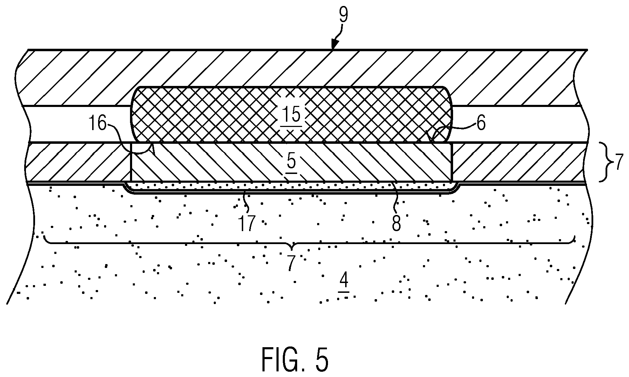

Figur 5 zeigt einen beispielhaften, schematischen Ausschnitt einer Querschnittsansicht im Bereich eines Durchleitungsabschnitts 5 des Unterbekleidungsstücks 1 in einer wie in Figur 3 gezeigten Tragesituation.

Hierbei wird das Unterbekleidungsstück 1 unter einem Elektromuskelstimulations-Kleidungsstück 9 und auf der Haut eines Trägers 4 getragen.

Wie man erkennen kann, liegt hierbei die Impulsübertragungsfläche 16 der gezeigten Muskelstimulationselektrode 15 flächig an der Elektroimpulsempfangsfläche 6 des Durchleitungsabschnitts 5 an. In Ausführungsformen, bei denen eine elektrisch leitfähige Beschichtung auf der Elektroimpulsempfangsfläche 6 aufgebracht ist, liegt entsprechend die Impulsübertragungsfläche 16 durch diese Beschichtung an der Elektroimpulsempfangsfläche 6 an. Die zugehörige Elektroimpulsabgabefläche 8 des Durchleitungsabschnitts 5 liegt wiederum flächig an der Haut des Trägers 4 an, in dieser Ausführungsform durch die Beschichtung 17.

Die Muskelstimulationselektrode 15 des EMS-Kleidungsstücks 9 ragt hier von dem umliegenden Textil des EMS-Bekleidungsstücks 9 etwas Richtung Unterbekleidungsstück 1 hervor, um ein bündiges Anliegen der Muskelstimulationselektrode 15 an die Elektroimpulsempfangsfläche 6 zu fördern. Aus diesem schematischen Aufbau wird weiterhin gut ersichtlich, dass durch das flächige Anliegen aller Komponenten und die vergleichsweise homogene Struktur des Unterbekleidungsmaterials 7, einschließlich des Durchleitungsabschnitts 5, Druckkräfte auf die Haut sehr gleichmäßig verteilt werden.

Nachfolgend wird kurz die Funktionsweise der zuvor beschriebenen möglichen Ausführung des Unterbekleidungsstücks 1 mit dem EMS-Kleidungsstück 9 erläutert.

Vor Beginn einer nicht-therapeutischen EMS-Anwendung zieht der Träger 4 das zuvor gereinigte Unterbekleidungsstück 1, hier das Oberkörperbekleidungsstück 2 und das Unterkörperbekleidungsstück 3, auf die nackte Haut an. Das Unterbekleidungsstück 1 kann hierzu beispielweise passend zu den körperlichen Gegebenheiten des Trägers 4 (z.B. Körpergröße) und den zu stimulierenden Muskelpartien ausgewählt werden. Durch die gezielte Anordnung der einzelnen Positionen der Durchleitungsabschnitte 5 passend zu einzelnen Muskelpartien des Trägers 4 kommen die Durchleitungsabschnitte 5 schnell und zuverlässig mit den passenden Hautstellen, durch die Beschichtung 17, in Kontakt.

Die hohe Flexibilität und Elastizität des Unterbekleidungsstücks 1, einschließlich an den Durchleitungsabschnitten 5, sorgt hierbei für ein verlässliches und Anliegen der jeweiligen Durchleitungsabschnitte 5 auf der Haut.

In einem anschließenden Schritt wird das EMS-Kleidungsstück 9 in gewohnter Weise über das Unterbekleidungsstück 1 angezogen und die einzelnen Gurte 14 für einen festen Sitz der Muskelstimulationselektroden 15 festgezogen. Hierbei kommt jede der Muskelstimulationselektroden 15 einfach und zuverlässig in einen flächigen und bündigen Kontakt mit der jeweiligen Elektroimpulsempfangsfläche 6 eines korrespondierenden Durchleitungsabschnitts, ohne das hierzu beispielsweise einzelne Verbindungen zwischen Durchleitungsabschnitten 5 und Muskelstimulationselektroden 15 des EMS-Kleidungsstücks 9 hergestellt werden müssten.

Weder die Haut des Trägers 4, noch das Unterbekleidungsstück 1 oder die Muskelstimulationselektroden 15 des EMS-Kleidungsstücks 9 müssen vor der Durchführung der EMS-Anwendung befeuchtet werden.

Anschließend wird eine gewünschte EMS-Anwendung mit dem Träger durchgeführt. Hierbei verhält sich das Unterbekleidungsstück 1 vergleichbar zu einer gewöhnlichen textilen Kleidung, wie beispielsweise Skiunterwäsche. Das Auftreten von Hautirritationen, Druck- oder Scheuerstellen verursacht durch das EMS-Kleidungsstück 9 wird vermieden und Körperschweiß wird zuverlässig aufgenommen.

Nach Beendigung der EMS-Anwendung wird zuerst das EMS-Kleidungsstück 9 abgelegt und anschließend das Unterbekleidungsstück 1.

Das Unterbekleidungsstück 1 kann danach in einfacher Weise, beispielsweise in einer Waschmaschine, gereinigt werden.

Das EMS-Kleidungsstück 9 steht aufgrund des Einsatzes des Unterbekleidungsstücks 1 sofort für eine nachfolgende EMS-Anwendung wieder zur Verfügung. Gegebenenfalls wird das EMS-Kleidungsstück 9 vor seinem Folgeeinsatz noch kurz in bekannter Weise, beispielsweise durch den Einsatz von Desinfektionsspray und Wischtüchern, oberflächlich gereinigt.

Die Betriebszeiten des EMS-Kleidungsstücks 9 werden durch den Einsatz des Unterbekleidungsstücks 1 erhöht. Der Tragekomfort für den EMS-Anwendungsempfänger 4 wird durch den Einsatz des Unterbekleidungsstücks 1 verbessert und das Anzieh- und Ausziehprozedere für Unterbekleidungsstück 1 und EMS-Kleidungsstück 9 verkürzt.

## Patentansprüche

1. Unterbekleidungsstück (1) zum Tragen unter einem Elektromuskelstimulations-Kleidungsstück (9), wobei
das Unterbekleidungsstück (1) mindestens einen sich durch das Unterbekleidungsmaterial (7) erstreckenden, elektrisch leitfähigen Durchleitungsabschnitt (5) aufweist, welcher eine Elektroimpulsempfangsfläche (6) auf der körperabgewandten Unterbekleidungsmaterial-Außenseite und eine Elektroimpulsabgabefläche (8) auf der körperzugewandten Unterbekleidungsmaterial-Innenseite umfasst, und der dazu eingerichtet ist, elektrische Impulse von der Elektroimpulsempfangsfläche (6) zur Elektroimpulsabgabefläche (8) zu leiten,
der mindestens eine Durchleitungsabschnitt (5) in dem Unterbekleidungsmaterial (7) flächig und einstückig integriert ist, und
das Unterbekleidungsmaterial (7) zumindest im Bereich des mindestens einen Durchleitungsabschnitts (5) und dem angrenzenden Unterbekleidungsmaterial (7) einen gemäß demselben Herstellungsverfahren gefertigten textilen Stoff aufweist, **dadurch gekennzeichnet dass**
der mindestens eine Durchleitungsabschnitt (5) in das Unterbekleidungsmaterial (7) eingenähtes elektrisch leitfähiges Garn aufweist, welches nach der ursprünglichen textilen Herstellung des Unterbekleidungsmaterials in das Unterbekleidungsmaterial eingenäht wurde.

2. Unterbekleidungsstück (1) nach Anspruch 1, **dadurch gekennzeichnet, dass**
sich die Elektroimpulsempfangsfläche (6) und die Elektroimpulsabgabefläche (8) in Größe und/oder in Materialbeschaffenheit einander entsprechen.

3. Unterbekleidungsstück (1) nach einem der vorherigen Ansprüche, **dadurch gekennzeichnet, dass**
die Elektroimpulsempfangsfläche (6) und/oder die Elektroimpulsabgabefläche (8) mit dem angrenzenden Unterbekleidungsmaterial oberflächenbündig sind, und/oder das Unterbekleidungsmaterial (7) zumindest im Bereich des mindestens einen Durchleitungsabschnitts (5) und dem angrenzenden Unterbekleidungsmaterial (7) einen konstanten Querschnitt aufweist.

4. Unterbekleidungsstück (1) nach einem der vorherigen Ansprüche, **dadurch gekennzeichnet, dass**
der oder die Durchleitungsabschnitte (5) so im Unterbekleidungsstück (1) angeordnet sind, dass die zugehörigen Elektroimpulsabgabeflächen (6) beim ordnungsgemäßen Tragen des Unterbekleidungsstücks (1) an einer oder mehreren Muskelpartien des Trägers sitzen, insbesondere an einer oder mehreren Muskelpartien der Skelettmuskulatur des Trägers sitzen, wie beispielsweise an Muskelpartien des Rückens, der Brust, der Beine, der Arme, des Bauchs und/oder der Schultern.

5. Unterbekleidungsstück (1) nach einem der vorherigen Ansprüche, **dadurch gekennzeichnet, dass**
der mindestens eine Durchleitungsabschnitt (5) eine vergleichbare Flexibilität wie das angrenzende Unterbekleidungsmaterial (7) aufweist.

6. Unterbekleidungsstück (1) nach einem der vorherigen Ansprüche, **dadurch gekennzeichnet, dass**
das Unterbekleidungsstück (1) ein Oberkörperbekleidungsstück (2) und/oder ein Unterkörperbekleidungsstück (3) aufweist, und/oder
das Unterbekleidungsstück (1) kabellos ist und/oder keine feste elektrische Kontaktstruktur aufweist, und/oder
der mindestens eine Durchleitungsabschnitt (5) als Trockenelektrode ausgeführt ist.

7. Unterbekleidungsstück (1) nach einem der vorherigen Ansprüche, **dadurch gekennzeichnet, dass** das Unterbekleidungsmaterial (7) durchgehend einen gemäß demselben Herstellungsverfahren gefertigten textilen Stoff aufweist.

8. Unterbekleidungsstück (1) nach einem der vorherigen Ansprüche, **dadurch gekennzeichnet, dass**
der mindestens eine Durchleitungsabschnitt (5) ein elektrisch leitfähiges Polymer, insbesondere ein elektrisch leitfähiges Silikon, aufweist, insbesondere in Kombination mit einem textilen Trägermaterial.

9. Unterbekleidungsstück (1) nach einem der vorherigen Ansprüche, **dadurch gekennzeichnet, dass**
die Elektroimpulsabgabefläche (8) und/oder die Elektroimpulsempfangsfläche (6) mit einer Beschichtung (17) aufweisend ein elektrisch leitfähiges Medium versehen sind, insbesondere mit einem elektrisch leitfähigen Polymer, beispielsweise einem elektrisch leitfähigen Silikon oder einem elektrisch leitfähigen Polyurethan.

10. Kombination aufweisend ein Unterbekleidungsstück (1) nach einem der Ansprüche 1 bis 9 und ein Elektromuskelstimulations-Kleidungsstück (9) mit mindestens einer Muskelstimulations-Elektrode (15).

11. Kombination nach Anspruch 10, **dadurch gekennzeichnet, dass**
die Position eines Durchleitungsabschnitts (5) des Unterbekleidungsstücks (1) und die Position einer Muskelstimulations-Elektrode (15) des Elektromuskelstimulations-Kleidungsstücks (9) so aufeinander abgestimmt sind, dass beim Tragen des Unterbekleidungsstücks (1) mit dem darüberliegenden Elektromuskelstimulations-Kleidungsstück (9) die Muskelstimulations-Elektrode (15) an einer Elektroimpulsempfangsfläche (6) des Durchleitungsabschnitts (5) anliegt, insbesondere jede Muskelstimulations-Elektrode (15) an einer Elektroimpulsempfangsfläche (6) anliegt.

12. Kombination nach Anspruch 10 oder 11, **dadurch gekennzeichnet, dass**
die flächige Ausdehnung der Elektroimpulsempfangsfläche (6) gleich groß oder größer als die flächige Ausdehnung der zugehörigen Muskelstimulations-Elektrode (15) ist.

13. Verwendung eines Unterbekleidungsstücks (1) nach einem der Ansprüche 1 bis 9 zum Tragen unter einem Elektromuskelstimulations-Kleidungsstück (9) während einer nicht-therapeutischen Elektromuskelstimulations-Anwendung.

14. Verwendung einer Kombination nach einem der Ansprüche 10 bis 12 während einer nicht-therapeutischen Elektromuskelstimulations-Anwendung.

## Claims

1. An undergarment (1) for wearing under an electrical muscle stimulation garment (9), wherein
the undergarment (1) includes at least one electrically conductive transmission portion (5) extending through the undergarment material (7), which comprises an electrical pulse receiving surface (6) on the outer side of the undergarment material facing away from the body and a an electrical pulse emitting surface (8) on the inner side of the undergarment material facing towards the body, and which is adapted to conduct electrical pulses from the electrical pulse receiving surface (6) to the electrical pulse emitting surface (8), wherein the at least one transmission portion (5) is in-plane integrally embedded in the undergarment material (7) and
the undergarment material (7) includes a textile material manufactured according to the same manufacturing process at least in the region of the at least one transmission portion (5) and the adjacent undergarment material (7), **characterized in that** the at least one transmission portion (5) includes electrically conductive yarn sewn into the undergarment material after the initial textile-making process for manufacturing the undergarment textile material.

2. The undergarment (1) according to claim 1, **characterized in that**
the electrical pulse receiving surface (6) and the electrical pulse emitting surface (8) correspond to each other in size and/or material properties.

3. The undergarment (1) according to any one of the preceding claims, **characterized in that**
the electrical pulse receiving surface (6) and/or the electrical pulse emitting surface (8) are flush with the surface of the adjacent undergarment material, and/or the undergarment material (7) has a constant cross-section at least in the region of the at least one transmission portion (5) and the adjacent undergarment material (7).

4. The undergarment (1) according to any one of the preceding claims, **characterized in that**
the one transmission portion or multiple transmission portions (5) are arranged in the undergarment (1) such that, when the undergarment (1) is properly worn, the associated electrical impulse emitting surfaces (6) are located on one or more muscle groups of the wearer, in particular on one or more muscle groups of the skeletal musculature of the wearer, such as muscle groups of the back, chest, legs, arms, abdomen and/or shoulders.

5. The undergarment (1) according to any one of the preceding claims, **characterized in that**
the at least one transmission portion (5) has flexibility comparable to that of the adjacent undergarment material (7).

6. The undergarment (1) according to any one of the preceding claims, **characterized in that**
the undergarment (1) comprises an upper body garment (2) and/or a lower body garment (3), and/or
the undergarment (1) is wireless and/or has no fixed electrical contact structure, and/or
the at least one transmission portion (5) is configured as a dry electrode.

7. The undergarment (1) according to any one of the preceding claims, **characterized in that** the undergarment material (7) includes a textile material made according to the same manufacturing process throughout.

8. The undergarment (1) according to any one of the preceding claims, **characterized in that**
the at least one transmission portion (5) includes an electrically conductive polymer, in particular an electrically conductive silicone, in particular in combination with a textile substrate material.

9. The undergarment (1) according to any one of the preceding claims, **characterized in that**
the electrical pulse emitting surface (8) and/or the electrical pulse receiving surface (6) are provided with a coating of an electrically conductive medium, in particular with an electrically conductive polymer, for example an electrically conductive silicone or an electrically conductive polyurethane.

10. A combination including an undergarment (1) according to anyone of claims 1 to 9 and an electrical muscle stimulation garment (9) having at least one muscle stimulation electrode (15).

11. The combination according to claim 10, **characterized in that**
the position of a transmission portion (5) of the undergarment (1) and the position of a muscle stimulation electrode (15) of the electrical muscle stimulation garment (9) are matched such that when the undergarment (1) with the overlying electrical muscle stimulation garment (9) is worn, the muscle stimulation electrode (15) abuts against an electrical impulse receiving surface (6) of the transmission portion (5), in particular each muscle stimulation electrode (15) abuts against an electrical pulse receiving surface (6).

12. The combination according to claims 10 or 11, **characterized in that**
the in-plane extent of the electrical impulse receiving surface (6) is equal to or greater than the in-plane extent of the associated muscle stimulation electrode (15).

13. A usage of the undergarment (1) according to anyone of claims 1 to 9 for wearing under an electrical muscle stimulation garment (9) during a non-therapeutic electrical muscle stimulation application.

14. A usage of a combination according to any one of claims 10 to 12 during a non-therapeutic electrical muscle stimulation application.

## Revendications

1. Sous-vêtement (1) destiné à être porté sous un vêtement d'électrostimulation musculaire (9),
le sous-vêtement (1) présentant au moins une section de passage (5) électriquement conductrice s'étendant à travers le matériau de sous-vêtement (7), qui comprend une surface de réception d'impulsions électriques (6) sur le côté extérieur du matériau de sous-vêtement tourné vers le corps et une surface d'émission d'impulsions électriques (8) sur le côté intérieur du matériau de sous-vêtement tourné vers le corps, et qui est conçu pour conduire des impulsions électriques de la surface de réception d'impulsions électriques (6) à la surface d'émission d'impulsions électriques (8),
l'au moins une section de conduction (5) étant intégrée à plat et d'un seul tenant dans le matériau de sous-vêtement (7), et
le matériau de sous-vêtement (7) présentant, au moins dans la zone de l'au moins une section de passage (5) et du matériau de sous-vêtement (7) adjacent, un tissu textile fabriqué selon le même procédé de fabrication, **caractérisé en ce que**
l'au moins une section de passage (5) présente un fil électriquement conducteur cousu dans le matériau de sous-vêtement (7), qui a été cousu dans le matériau de sous-vêtement après la fabrication textile initiale du matériau de sous-vêtement.

2. Sous-vêtement (1) selon la revendication 1, **caractérisé en ce que**
la surface de réception d'impulsions électriques (6) et la surface d'émission d'impulsions électriques (8) correspondent l'une à l'autre en termes de taille et/ou de nature de matériau.

3. Sous-vêtement (1) selon l'une quelconque des revendications précédentes, **caractérisé en ce que**
la surface de réception d'impulsions électriques (6) et/ou la surface d'émission d'impulsions électriques (8) affleurent à la surface avec le matériau de sous-vêtement adjacent, et/ou le matériau de sous-vêtement (7) présente une section transversale constante au moins dans la zone de la au moins une section de passage (5) et du matériau de sous-vêtement adjacent (7).

4. Sous-vêtement (1) selon l'une quelconque des revendications précédentes, **caractérisé en ce que**
le ou les sections de passage (5) sont disposées dans le sous-vêtement (1) de telle sorte que, lorsque le sous-vêtement (1) est correctement porté, les surfaces d'émission d'impulsions électriques associées (8) sont situées sur une ou plusieurs parties musculaires de l'utilisateur, en particulier sur une ou plusieurs parties musculaires de la musculature squelettique de l'utilisateur, telles que des parties musculaires du dos, de la poitrine, des jambes, des bras, de l'abdomen et/ou des épaules.

5. Sous-vêtement (1) selon l'une quelconque des revendications précédentes, **caractérisé en ce que**
l'au moins une section de passage (5) présente un matériau de sous-vêtement comparable au matériau de sous-vêtement (7) adjacent.

6. Sous-vêtement (1) selon l'une quelconque des revendications précédentes, **caractérisé en ce que**
le sous-vêtement (1) présente un vêtement pour le torse (2) et/ou un vêtement pour le bas du corps (3), et/ou
que le sous-vêtement (1) est sans fil et/ou ne présente pas de structure de contact électrique fixe, et/ou
l'au moins une section de conduction (5) est réalisée sous la forme d'une électrode sèche.

7. Sous-vêtement (1) selon l'une quelconque des revendications précédentes, **caractérisé en ce que**
le matériau de sous-vêtement (7) présente en continu un tissu textile fabriqué selon le même procédé de fabrication.

8. Sous-vêtement (1) selon l'une quelconque des revendications précédentes, **caractérisé en ce que**
l'au moins une section de passage (5) comprend un polymère électriquement conducteur, en particulier un silicone électriquement conducteur, en particulier en combinaison avec un matériau de support textile.

9. Sous-vêtement (1) selon l'une quelconque des revendications précédentes, **caractérisé en ce que**
la surface d'émission d'impulsions électriques (8) et/ou la surface de réception d'impulsions électriques (6) est munie d'un revêtement (17) présentant un milieu électriquement conducteur, en particulier d'un polymère électriquement conducteur, par exemple d'un silicone électriquement conducteur ou d'un polyuréthane électriquement conducteur.

10. Combinaison comprenant un sous-vêtement (1) selon l'une quelconque des revendications 1 à 9 et un vêtement d'électrostimulation musculaire (9) avec au moins une électrode de stimulation musculaire (15).

11. Combinaison selon la revendication 10, **caractérisée en ce que** lorsque le sous-vêtement (1) est porté avec le vêtement (9) de stimulation électrique des muscles qui le recouvre, la position d'une section de passage (5) du sous-vêtement (1) et la position d'une électrode (15) de stimulation des muscles du vêtement d'électrostimulation musculaire (9) sont adaptées l'une à l'autre de telle sorte que l'électrode de stimulation musculaire (15) s'applique contre une surface (6) de réception des impulsions électriques de la section de passage (5), en particulier chaque électrode (15) de stimulation des muscles s'applique contre une surface (6) de réception des impulsions électriques.

12. Combinaison selon la revendication 10 ou 11**, caractérisée en ce que** l'étendue en surface de la surface de réception d'impulsions électriques (6) est supérieure ou égale à l'étendue en surface de l'électrode de stimulation musculaire (15) correspondante.

13. Utilisation d'un sous-vêtement (1) selon l'une quelconque des revendications 1 à 9 destiné à être porté sous un vêtement d'électrostimulation musculaire (9) pendant une administration d'électrostimulation musculaire non thérapeutique.

14. Utilisation d'une combinaison selon l'une quelconque des revendications 10 à 12 durant une administration d'électrostimulation musculaire non thérapeutique.
